# EUROPEAN PATENT SPECIFICATION

(11) **EP 0 338 565 B1**
(45) Date of publication and mention of the grant of the patent: **10.08.1994**
(21) Application number: 89107155.7
(22) Date of filing: 20.04.1989
(51) Int. Cl.: C07C 233/16, A61K 7/48, A61K 31/16

(54) **N-Alkoxyalkylamides of hydroxyacids and skin treating compositions therewith**
N-Alkoxyalkylamide von Hydroxysauren und sie enthaltende Hautbehandlungszubereitungen
N-alkoxyalklamides d'hydroxyacides et compositions de ces derniers pour le traitement de la peau

(30) Priority: 22.04.1988 US 184858
(43) Date of publication of application: 25.10.1989
(73) Proprietor: Revlon Consumer Products Corporation, New York, NY 10022 (US)
(72) Inventor: Ciaudelli, Joseph P., Ramsey, NJ (US)
(74) Representative: Sanderson, Laurence Andrew

(56) References cited:
- DE-A- 2 321 752
- DE-A- 2 338 087
- DE-A- 2 632 391
- US-A- 3 322 635
- CHEMICAL ABSTRACTS, vol. 63, no. 9, 25 October 1965, Columbus, OH (US); H. RUEBNER, no. 11339h#
- CHEMICAL ABSTRACTS, vol. 33, no. 18, 20 September 1939, Columbus, OH (US); K.J. GOLDNER et al., no. 7278#

## Description

The present invention relates to skin-care compositions containing certain N-alkoxyalkylamides of hydroxyacids, which can be used for moisturizing and/or softening human skin, particularly so as to moderate so-called "dry skin".

The human skin disorder known as "dry skin" is characterized by cracking, flaking or scaling of the skin of the hands, feet, neck, face or other parts of the body. This disorder may result from a hereditary condition known as ichthyosis, which is a severe form of dry skin - but fortunately this severe form of dry skin is not too prevalent. The more common form of "dry skin", which affects a relatively large proportion of the population, is a mild to moderate condition which arises from exposure to environmental conditions of low humidity in the autumn and winter seasons of the temperate climatic zones, and it is primarily this kind of dry skin caused by environmental conditions with which we are here concerned - arising from a loss of moisture from skin areas exposed to the climatic conditions, with attendant formation of fissures, chaps, cracks or flakes in the affected skin areas.

Over the years, a variety of chemical compounds have been proposed for use in combating such dry skin problems, these compounds being normally formulated with other materials so as to be useful for topical application to the skin in the form of a lotion, cream or ointment.

Examples of such prior art compounds, and the topical compositions in which they may be used for treating dry skin, are for example disclose in US-A-3,230,228, US-A-3,322,635, US-A 4,105,783, US-A 4,197,316, and US-A 4,382,765, as well as also in DE-A-2,732,391, and the compounds disclosed in these references do include some hydroxy-containing carboxylic acid amides.

We have however now found that certain particular N-alkoxyalkyamide compounds hereinafter identified are especially effective in the prevention or treatment of at least the commoner forms of dry skin.

According to this invention there is provided a skin-care composition for topical application to human skin comprising a skin softening and moisturizing effective amount of one or more N-alkoxyalkylamide compounds of the general formula: 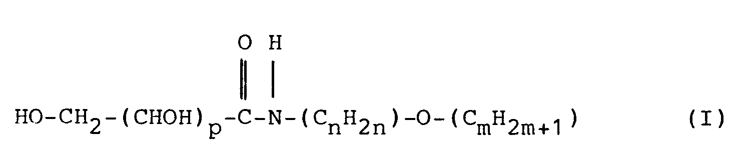
(wherein p is an integer from 1 to 4, -(CₙH₂ₙ)- is a straight- or branched-chain alkylene linkage in which n is an integer of 1 to 6, and -(CₘH₂ₘ₊) is a straight- or branched-chain alkyl group in which m is an integer of 1 to 6) together with a cosmetically-acceptable topical carrier.

The compositions of the present invention are designed for use in treating humans having a dry skin condition. The active ingredient used in such compositions for such purposes is one or more of the N-alkoxyalkylamide compounds disclosed above, of general formula I above.

Neither the N-alkoxyalkylamide compounds themselves nor the compositions of the present invention containing them are irritating to the skin, allergenic or toxic.

The compositions of the present invention may be used for not only prophylactic but also therapeutic purposes, relative to their proposed use in treating dry skin by topical application thereto, so as to prevent or cure the occurrence of any cracking, flaking, scaling or chapping of the skin. Thus, the compositions of the present invention may be used to prevent, cure or ameliorate dry skin conditions, as well as acne, psoriasis, seborrhea, keratosis, diaper rash, sunburn and windburn.

This invention from another aspect is therefore also concerned with the use of one or more of the N-alkoxyalkylamide compounds of the general formula I for the manufacture of a medicament for the prevention, amelioration or cure of dry skin conditions and/or of acne, psoriasis, seborrhea, keratosis, diaper rash, sunburn and windburn.

The compositions of the present invention may be prepared and used in the form of a lotion, cream, ointment, stick or soap, or other forms commonly employed in the art for skin-care formulations. They are preferably used in an emulsified form. The compositions will normally comprise from 1 to 20% by weight of the compound(s) of general formula I.

In the compositions of this invention it is preferred to employ one or more of the compounds of general formula I in which p is 4, and/or those in which n is 3, and/or m is 1. Thus, the specific preferred N-alkoxyalkylamide of general formula I for use in the compositions of this invention is that wherein p is 4, n is 3 and m is 1, namely N-methoxypropylgluconamide: 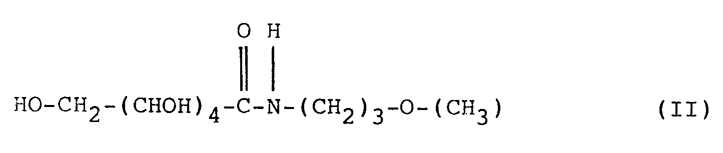
The compositions of the present invention are prepared employing skin-softening and -moisturizing effective amounts of one or more of the N-alkoxyalkylamide compounds of the present invention in a cosmetically-acceptable carrier, such as a hydrophilic ointment(USP) or petrolatum. When used in such compositions it is preferred that they should contain 1 to 20%, and preferably 5 to 15%, by weight of the N-alkoxyalkylamide compound(s) therein.

The bulk of the compositions of the present invention will comprise 50 to 75%, and preferably 55 to 65%, by weight of distilled water, and 10 to 40%, and preferably 15 to 30%, by weight of a combination of other commonly-used cosmetically-effective auxiliary components of the various types of composition in question, i.e. dependent on whether formulated as lotion, cream, ointment, stick, soap or otherwise. The auxiliary components chosen for use in such compositions must be chemically-inert with respect to each other, and with respect to the N-alkoxyalkylamide compounds of the general formula I.

The auxiliary components used in the compositions of the present invention, in addition to water, would include the following amounts (expressed in % by weight based on the total weight of the final composition) of the following types of additives:
- 0.75 to 7.00 % by weight of emulsifying agent;
- 3.00 to 15.00 % by weight of emollients;
- 1.00 to 20.00 % by weight of one or more N-alkoxyalkylamides of the general formula I given herein;
- 0.10 to 5.00 % by weight of lubricant;
- 0.20 to 1.00 % by weight of preservative;
- 0.20 to 1.00 % by weight of perfume; and
- 0.01 to 0.10 % by weight of colorant;
the remainder being water.

Lists of such materials, which are well known in the art, are disclosed for example in:
"Cosmetics: Science and Technology," Edited by M.S. Balsam and E. Sagarin, 2nd Edition, 1972, Wiley Pub. Co.;
"The Chemistry and Manufacture of Cosmetics" by M.G. Denavasse; and
"Harry's Cosmeticology," J.B. Wilkinson et al., 7th Edition, 1982, Chem. Pub. Co.

The compositions of this invention, when used for treating dry skin, may be topically applied one or more times a day, and preferably 2 to 4 times per day, to the area of skin to be treated therewith, the period of treatment extending over 7 to 21 days, in order to achieve the desired amelioration of the dry skin condition.

Certain of the N-alkoxyalkylamide compounds of the general formula I above have already been disclosed as such, see for instance DE-A-23 38 087.

However, the specific preferred N-alkoxyalkylamide for use in the compositions of this invention, namely N-methoxypropylgluconamide, is new, and therefore as such it forms part of this invention.

Both the already-known and the previously-unknown N-alkoxyalkylamides of general formula I may be prepared under amide-forming conditions by reacting an amine compound having the general formula:

H₂N-(CₙH₂ₙ)-O-(CₘH₂ₘ₊₁) (III)

[wherein -(CₙH₂ₙ)- and -(CₘH₂ₘ₊₁) are as defined above] either with a carboxylic acid having the general formula: 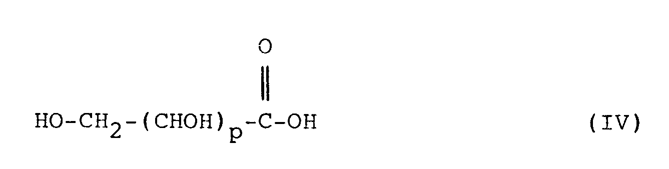
(in which p as before is an integer from 1 to 4) or with a lactone having the general formula: 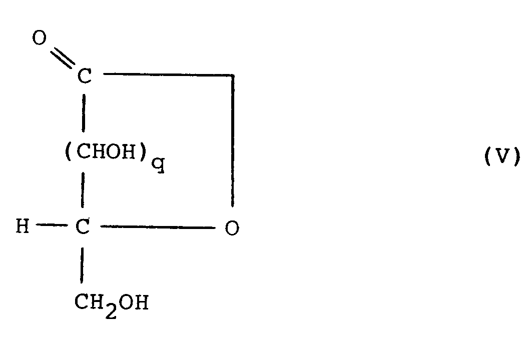
in which q is an integer from 1 to 3.

The preferred amine of general formula III for use as starting material in this process is methoxypropylamine, i.e. wherein m is 1 and n is 3, while the preferred carboxylic acid of general formula IV is gluconic acid in which p is 4 or alternatively the preferred lactone of general formula V is glucono-delta-lactone in which q is 3.

In carrying out the reaction, one mole of the amine of general formula III is actually reacted with one mole of the acid/lactone of general formula IV/V, but in order to help drive the reaction to completion an excess of the amine can advantageously be used. A suitable such excess is usually in the amount of 0.1 to 0.5 mole %.

The lactone/acid is first heated with stirring in a refluxing alcoholic solvent, at atmospheric pressure, so as to slurry the solids. The alcohol used is aliphatic, and has a reflux temperature of 65 to 86°C. Then the amine is added dropwise to the slurry, in the refluxing solvent, and the solids therein are dissolved to produce a light yellow-coloured solution. The reaction takes 10 to 30 minutes at reflux temperatures. The reaction is slightly exothermic. Any temperature rise is however only noticeable when the amine is added at room temperature, but is not detectable at solvent-reflux temperatures. The heating is then discontinued, and the solution is allowed to cool with stirring. A precipitate generally starts to form approximately 20°C below the refluxing temperature of the solvent. Upon reaching room temperature (25°C), the solids are then filtered off using a Buchner funnel under vacuum. The solids are washed with alcohol solvent. Yields of 90 to 98% after drying are thus obtained.

The N-alkoxyalkylamide compounds of the general formula I thus obtained may be stored in closed containers, either as is or in the form of the compositions disclosed herein, for extended periods of time at room temperature without any change in their utility for dry skin treating purposes.

The following examples are merely illustrative of various aspects of the present invention.

### Example 1

Glucono delta lactone (352 grams) was heated in 1000 ml of isopropyl alcohol to reflux temperatures, about 86°C (at atmospheric pressure), in a two-liter 3-neck glass flask equipped with a stirrer, a reflux condenser column and a stoppered inlet port. Then over a period of about 10 minutes 180 grams of methoxypropylamine was added through the inlet port to the refluxing lactone slurry.

The resulting system was then stirred and heated at reflux (86°C) for an additional 10 minutes, and then the heat was turned off. At this temperature (∼86°C) all the components of the system were dissolved in the isopropyl alcohol. As the system cooled a precipitate started to form at approximately 65°C.

When the temperature of the system reached ambient temperature (∼25°C), the system was filtered through a Buchner funnel under vacuum (house) and the filtrate was recovered. The solid was transferred to a pyrex dish for air drying (over about 6 hours). A yield of 510 grams of product (methoxy propyl gluconamide) was thus obtained, for a yield of 95.86% of the theoretical.

An additional amount of such product, 12 grams, was also obtained after distilling off ∼90% of the isopropyl alcohol from the filtrate that passed through the filter.

The two portions of the recovered solids were blended together and about 100 grams of the thus recovered solids were recrystallized in water and isopropyl alcohol.

An analysis of such product showed that it contained 5.17% nitrogen, as compared to 5.18% theoretical. The compound had a melting point of 107.5°C. The IR spectrum of the compound showed significant bands at 3500, 3400, 3340, 2920, 2880, 1650, 1535, 1430, 1240, 1180, 1095, 1070, 1035, 730 and 630 reciprocal centimeters (cm⁻¹). These bands are characteristic of hydroxy stretch, nitrogen-hydrogen stretch, methyl stretch, methoxy stretch and various secondary amide bands.

### Examples 2 to 3

Two cream formulations (in 1 kilogram batches each) were prepared to comparatively evaluate the compound made in Example 1 as a skin moisturizing or softening material. The creams were emulsions prepared as described below. These two formulations, in weight %, were as follows:

| Component | Weight % of Component | |
|---|---|---|
| | Example 2 | 3 |
| Water | 70.36 | 70.36 |
| Propylene Glycol | 1.00 | 1.00 |
| Methyl p-Hydroxybenzoate | 0.30 | 0.30 |
| Mineral Oil | 1.50 | 1.50 |
| C₁₂₋₁₅ Alcohol Benzoate | 1.50 | 1.50 |
| Glyceryl Monostearate | 2.30 | 2.30 |
| Polawax* | 4.08 | 4.08 |
| Stearyl Alcohol | 1.50 | 1.50 |
| Polyoxethylene 21 Stearyl Ether | 0.75 | 0.75 |
| Silicone Oil | 0.21 | 0.21 |
| Stearyl Stearoyl Stearate | 1.00 | 1.00 |
| Propyl p-Hydroxybenzoate | 0.10 | 0.10 |
| Bisabolol | 0.20 | 0.20 |
| Imidazolidinyl Urea | 0.20 | 0.20 |
| Methoxy Propyl Gluconamide | -- | 15.00 |
| Glucono Delta Lactone | 15.00 | -- |
| TOTAL | 1̅0̅0̅.̅0̅0̅ | 1̅0̅0̅.̅0̅0̅ |

| | | |
|---|---|---|
| * Polawax (Croda, Inc.) is a preparation of higher fatty alcohols and ethylene oxide reaction products. | | |

Both emulsions were prepared from four (4) sub-combinations, or phases, of the components listed above.

Phase A is made of 52.36% water, 1.00% propylene glycol and 0.30% methyl-p-hydroxy benzoate.

Phase D is made of 15.00% water and 15.00% of the additive being comparatively evaluated, i.e., methoxy propyl gluconamide or glucono delta lactone.

Phase B contains all the remaining components except the imidazolidinyl urea.

The components of Phase A are first mixed together with heating at 80°C, and the components of Phase B are also mixed together at 80°C. Then Phase B is added to Phase A and heating (at 80°C) and mixing is continued for about 10 minutes and then the heating is stopped.

Phase C is formed by dissolving the imidazolidinyl urea in the water and heating to 50°C. Phase C is then added to the Phase A/B admixture. Phase D is made by dissolving the additive of choice in water and heating to 45°C, then Phase D is added to the Phase A/B/C admixture. The resulting product is then cooled to 35°C with mixing and packaged.

The formulations of Examples 2 to 3 were comparatively evaluated by a test panel of 10 panelists with dry skin.

In evaluating the test formulations, the panelists cleansed both of their forearms with their regular soap once in the morning and once in the evening and than applied the test formulation to one forearm. The other forearm was left untreated as a control. Each formulation was thus tested twice daily for a two week period. At the end of this time, the forearms of each panelist were compared. The results showed that although the formulation of Example 2 provided some amelioration of the dry skin condition of the treated forearms, as compared to the untreated forearms, the use of the formulation of Example 3 provided a noticeably improved difference in its effect on the dry skin of the forearms treated therewith as compared to the effect provided by the use of the formulation of Example 2.

### Example 4

This example illustrates the compatibility of methoxy propyl gluconamide with other cosmetic ingredients including sunscreening agents.

| Component | Weight % |
|---|---|
| Water | 62.125 |
| Methyl Paraben | 0.250 |
| Propylene Glycol | 5.000 |
| Carbomer 941 | 0.125 |
| Triethanolamine | 0.100 |
| Glyceryl Stearate and Laureth-23 (polyoxyethylene ether) | 8.000 |
| Cetyl Alcohol | 1.500 |
| Methyl Paraben | 0.150 |
| Butylated Hydroxy Anisole | 0.150 |
| C₁₂₋₁₅ Alcohol Benzoate | 5.000 |
| Ethyl Hexyl Linoleoyl Oxystearate | 5.000 |
| Bisabolol | 0.200 |
| Glycol Stearate | 3.000 |
| Dimethicone | 1.000 |
| Polyoxyethylene 21 Stearyl Ether | 0.750 |
| Octyl Dimethyl PABA | 3.000 |
| Benzophenone-3 | 1.500 |
| Dimethylol Dimethyl Hydantoin | 0.400 |
| Perfume | 0.250 |
| Methoxy Propyl Gluconamide | 2.500 |
| TOTAL | 1̅0̅0̅.̅0̅0̅0̅ |

The methoxy propyl gluconamide was physically, chemically and functionally compatible with the other components of the Example 4 formulation. The use of the methoxy propyl gluconamide in the Example 4 formulation provides noticeably improved dry skin treating properties as compared to the use of the same formulation without such amide therein. The formulation is also useful for sunscreening purposes.

### Example 5

The following illustrates the compatibility of the subject amide compound in a water in oil emulsion formulation.

| Component | Weight % |
|---|---|
| Water | 52.058 |
| Methyl Paraben | 0.200 |
| Ethyl Paraben | 0.150 |
| Disodium Salt of Ethylene Diamine Tetraacetic Acid | 0.240 |
| Butylated Hydroxy Anisole | 0.150 |
| Propylene Glycol | 4.000 |
| Triethanolamine | 0.092 |
| Cyclomethicone | 10.000 |
| Carbopol 1342 (Carbomer 1342) | 0.110 |
| Dimethicone 50 cs | 10.000 |
| Dimethicone 1000 cs | 7.500 |
| Isocetyl Linoleoyl Oxystearate | 5.000 |
| *Abil WS08 | 5.000 |
| Phenoxyethanol | 0.500 |
| Methoxy Propyl Gluconamide | 5.000 |
| TOTAL | 1̅0̅0̅.̅0̅0̅0̅ |

| | |
|---|---|
| * Abil WS08 (Goldschmidt AG) is a combination of Cetyl Dimethicone Copolyol Cethyl Dimethicone, Polyglyceryl-3 Oleate and Hexyl Laurate. | |

The methoxy propyl gluconamide was physically, chemically and functionally compatible with the other components of the Example 5 formulation. The use of the methyoxy propyl gluconamide in the Example 5 formulation provides noticeably improved dry skin treating properties as compared to the use, for such purposes, of the same formulations without such amide therein.

## Claims (Claims for the following Contracting State(s): AT, BE, CH, LI, DE, FR, GB, IT, LU, NL, SE)

1. A skin-care composition for topical application to human skin comprising a skin softening and moisturizing effective amount of one or more N-alkoxyalkylamide compounds of the general formula: 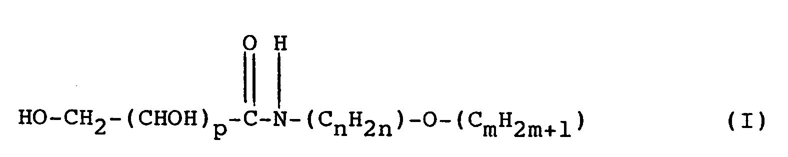 (wherein p is an integer from 1 to 4, (CₙH₂ₙ) is a straight- or branched-chain alkylene linkage in which n is an integer of 1 to 6, and (CₘH₂ₘ₊₁) is a straight- or branched-chain alkyl group in which m is an integer of 1 to 6) together with a cosmetically-acceptable topical carrier.

2. A composition as claimed in claim 1, which comprises from 1 to 20% by weight of the compound(s) of general formula I with a topical carrier such as to form a lotion, cream, ointment, stick or soap.

3. A composition as claimed in claim 1 or claim 2, including one or more of the compounds of general formula I in which p is 4.

4. A composition as claimed in claim 1 or claim 2, including one or more of the compounds of general formula I in which n is 3.

5. A composition as claimed in claim 1 or claim 2, including one or more of the compounds of general formula I in which m is 1.

6. A composition as claimed in any of claims 1 to 5, including one or more of the compounds of general formula I in which p is 4, n is 3 and m is 1.

7. A composition as claimed in claim 6, including N-methoxy propyl gluconamide.

8. A composition as claimed in any of the preceding claims, which contains from 5% to 15% by weight of the compound(s) of general formula I.

9. A composition as claimed in any of the preceding claims, which besides the compound(s) of general formula I and water also contains from 10% to 40% by weight of a combination of chemically-inert cosmetically-effective auxiliary components appropriate to the type of composition.

10. A composition as claimed in any of the preceding claims, which is in the form of an emulsion.

11. A composition as claimed in claim 10, which contains one or more compounds of general formula I together with:
- from 0.75% to 7.00% by weight of one or more emulsifying agent(s);
- from 3.00% to 15.00% by weight of one or more emollient(s);
- from 0.10% to 5.00% by weight of one or more lubricant(s);
- from 0.20% to 1.00% by weight of one or more preservative(s);
- from 0.20% to 1.00% by weight of perfume; and/or
- from 0.01% to 0.10% by weight of one or more colorant(s), the balance being water.

12. Use of one or more N-alkoxyalkylamide compounds of the general formula: 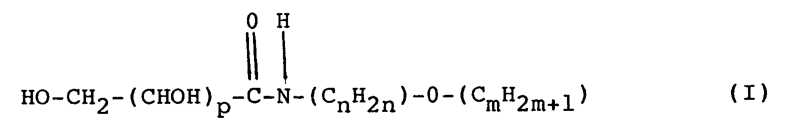 (wherein p is an integer from 1 to 4, (CₙH₂ₙ) is a straight- or branched-chain alkylene linkage in which n is an integer of 1 to 6, and (CₘH₂ₘ₊₁) is a straight- or branched-chain alkyl group in which m is an integer of 1 to 6) for the manufacture of a composition for the prevention, amelioration or cure of dry skin conditions and/or acne, psoriasis, seborrhea, keratosis, diaper rash, sunburn and windburn.

13. Use according to claim 12, for the manufacture of a composition for moisturizing and softening human skin which is a lotion, cream, ointment, stick or soap.

14. Use as claimed in claim 12 or claim 13, in which the compound of general formula I employed is or includes methoxy propyl gluconamide.

15. Methoxy propyl gluconamide.

## Claims (Claims for the following Contracting State(s): ES)

1. A method for the manufacture of a cosmetic preparation for topical application to human skin in which one incorporates one or more N-alkoxyalkylamide compounds of the general formula: 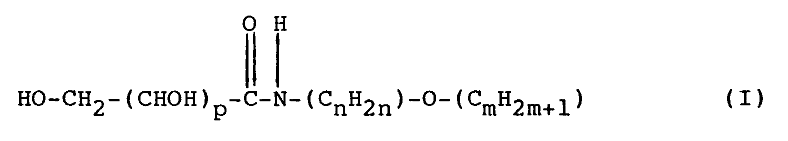 (wherein p is an integer from 1 to 4, (CₙH₂ₙ) is a straight- or branched-chain alkylene linkage in which n is an integer of 1 to 6, and (CₘH₂ₘ₊₁) is a straight- or branched-chain alkyl group in which m is an integer of 1 to 6) into a cosmetically-acceptable topical carrier in an amount sufficient to impart a skin-softening and -moisturizing effect to the resultant cosmetic preparation.

2. A method as claimed in claim 1, in which one incorporates from 1 to 20% by weight of the compound(s) of general formula I into such a topical carrier as to form a lotion, cream, ointment, stick or soap.

3. A method as claimed in claim 1 or claim 2, in which one incorporates one or more of the compounds of general formula I in which p is 4.

4. A method as claimed in claim 1 or claim 2, in which one incorporates one or more of the compounds of general formula I in which n is 3.

5. A method as claimed in claim 1 or claim 2, in which one incorporates one or more of the compounds of general formula I in which m is 1.

6. A method as claimed in any of claims 1 to 5, in which one incorporates one or more of the compounds of general formula I in which p is 4, n is 3 and m is 1.

7. A method as claimed in claim 6, in which one incorporates N-methoxy propyl gluconamide.

8. A method as claimed in any of the preceding claims, in which one incorporates from 5% to 15% by weight of the compound(s) of general formula I.

9. A method as claimed in any of the preceding claims, in which the cosmetically-acceptable topical carrier contains water and from 10% to 40% by weight of a combination of chemically-inert cosmetically-effective auxiliary components appropriate to the type of composition desired.

10. A method as claimed in any of the preceding claims, in which the cosmetic preparation thus manufactured takes the form of an emulsion.

11. A method as claimed in claim 10, in which the compound(s) of general formula I are incorporated in a carrier which contains:
- from 0.75% to 7.00% by weight of one or more emulsifying agent(s);
- from 3.00% to 15.00% by weight of one or more emollient(s);
- from 0.10% to 5.00% by weight of one or more lubricant(s);
- from 0.20% to 1.00% by weight of one or more preservative(s);
- from 0.20% to 1.00% by weight of perfume; and/or
- from 0.01% to 0.10% by weight of one or more colorant(s), the balance being water.

12. A method as claimed in any of the preceding claims, which includes the preliminary step of preparing the N-alkoxyalkylamide compound(s) of general formula I for incorporation into the carrier by reacting an amine of general formula:
H₂N-(CₙH₂ₙ)-O-(CₘH₂ₘ₊₁) (III)
(wherein -(CₙH₂ₙ)- is a straight- or branched-chain alkylene linkage in which n is an integer of 1 to 6, and -(CₘH₂ₘ₊₁) is a straight- or branched-chain alkyl group in which m is an integer of 1 to 6) under amide-forming conditions either with a carboxylic acid having the general formula: 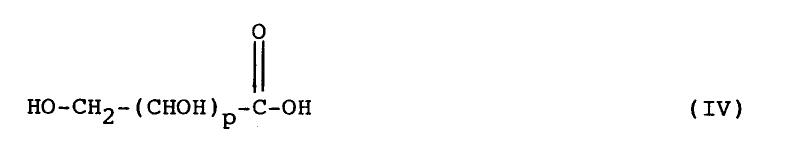 (in which p is an integer from 1 to 4) or with a lactone having the general formula: 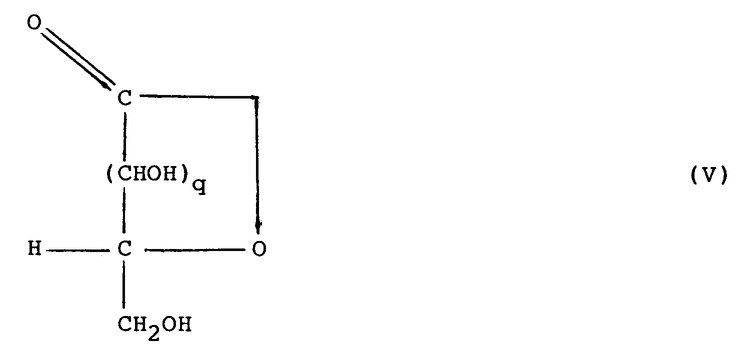 (in which q is an integer from 1 to 3) and thereafter incorporating the desired product into the carrier.

13. A method as claimed in claim 12, in which the amine of general formula III employed is methoxypropylamine.

14. A method as claimed in claim 12 or claim 13, in which the carboxylic acid of general formula IV employed is gluconic acid.

15. A method as claimed in claim 12 or claim 13, in which the lactone of general formula V employed is glucono-delta-lactone.

16. A process for the production of N-methoxy-propyl-gluconamide in which one reacts methoxy-propyl-amine under amide-forming conditions either with gluconic acid or with glucono-delta-lactone.

17. A process as claimed in claim 16, in which a slight molar excess of the methoxy-propyl-amine is reacted with glucono-delta-lactone in solution in an aliphatic alcohol refluxing at atmospheric pressure.

## Claims (Claims for the following Contracting State(s): GR)

1. A skin-care composition for topical application to human skin comprising a skin softening and moisturizing effective amount of one or more N-alkoxyalkylamide compounds of the general formula: 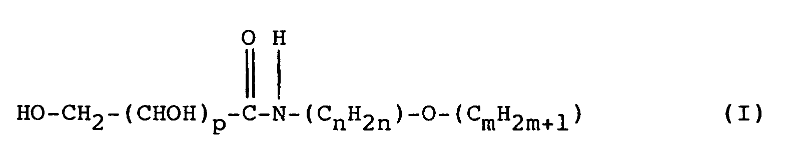 (wherein p is an integer from 1 to 4, (CₙH₂ₙ) is a straight- or branched-chain alkylene linkage in which n is an integer of 1 to 6, and (CₘH₂ₘ₊₁) is a straight- or branched-chain alkyl group in which m is an integer of 1 to 6) together with a cosmetically-acceptable topical carrier.

2. A composition as claimed in claim 1, which comprises from 1 to 20% by weight of the compound(s) of general formula I with a topical carrier such as to form a lotion, cream, ointment, stick or soap.

3. A composition as claimed in claim 1 or claim 2, including one or more of the compounds of general formula I in which p is 4.

4. A composition as claimed in claim 1 or claim 2, including one or more of the compounds of general formula I in which n is 3.

5. A composition as claimed in claim 1 or claim 2, including one or more of the compounds of general formula I in which m is 1.

6. A composition as claimed in any of claims 1 to 5, including one or more of the compounds of general formula I in which p is 4, n is 3 and m is 1.

7. A composition as claimed in claim 6, including N-methoxy propyl gluconamide.

8. A composition as claimed in any of the preceding claims, which contains from 5% to 15% by weight of the compound(s) of general formula I.

9. A composition as claimed in any of the preceding claims, which besides the compound(s) of general formula I and water also contains from 10% to 40% by weight of a combination of chemically-inert cosmetically-effective auxiliary components appropriate to the type of composition.

10. A composition as claimed in any of the preceding claims, which is in the form of an emulsion.

11. A composition as claimed in claim 10, which contains one or more compounds of general formula I together with:
- from 0.75% to 7.00% by weight of one or more emulsifying agent(s);
- from 3.00% to 15.00% by weight of one or more emollient(s);
- from 0.10% to 5.00% by weight of one or more lubricant(s);
- from 0.20% to 1.00% by weight of one or more preservative(s);
- from 0.20% to 1.00% by weight of perfume; and/or
- from 0.01% to 0.10% by weight of one or more colorant(s), the balance being water.

12. A method for the manufacture of a cosmetic preparation for topical application to human skin as claimed in any of claims 1 to 11, in which one incorporates one or more N-alkoxyalkylamide compounds of the general formula: 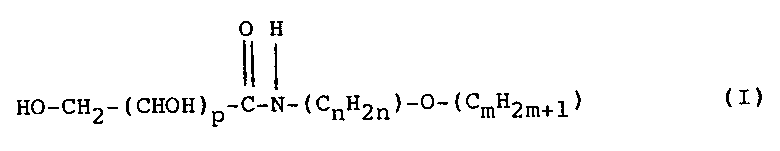 (wherein p is an integer from 1 to 4, (CₙH₂ₙ) is a straight- or branched-chain alkylene linkage in which n is an integer of 1 to 6, and (CₘH₂ₘ₊₁) is a straight- or branched-chain alkyl group in which m is an integer of 1 to 6) into a cosmetically-acceptable topical carrier in an amount sufficient to impart a skin-softening and -moisturizing effect to the resultant cosmetic preparation.

13. A method as claimed in claim 12, which includes the preliminary step of preparing the N-alkoxyalkylamide compound(s) of general formula I for incorporating into the carrier by reacting an amine of general formula:
H₂N-(CₙH₂ₙ)-O-(CₘH₂ₘ₊₁) (III)
[wherein -(CₙH₂ₙ)- and -(CₘH₂ₘ₊₁) are a previously defined] under amide-forming conditions either with a carboxylic acid having the general formula: 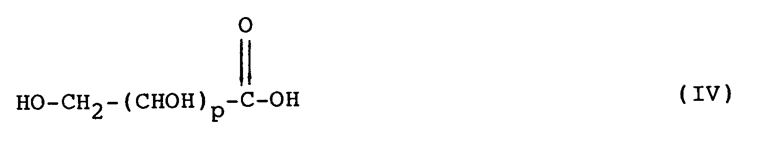 (in which p is an integer from 1 to 4) or with a lactone having the general formula: 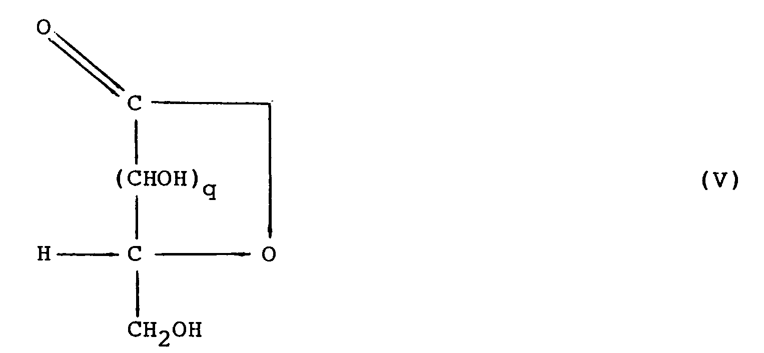 (in which q is an integer from 1 to 3).

14. A method as claimed in claim 13, in which the amine of general formula III employed is methoxypropylamine.

15. A method as claimed in claim 13 or claim 14, in which the carboxylic acid of general formula IV employed is gluconic acid.

16. A method as claimed in claim 13 or claim 14, in which the lactone of general formula V employed is glucono-delta-lactone.

17. A process for the production of N-methoxy-propyl-gluconamide in which one reacts methoxy-propyl-amine under amide-forming conditions either with gluconic acid or with glucono-delta-lactone.

18. A process as claimed in claim 17, in which a slight molar excess of the methoxy-propyl-amine is reacted with glucono-delta-lactone in solution in an aliphatic alcohol refluxing at atmospheric pressure

## Patentansprüche (Patentansprüche für folgende(n) Vertragsstaat(en): AT, BE, CH, LI, DE, FR, GB, IT, LU, NL, SE)

1. Hautpflege-Zusammensetzung zur äußeren Anwendung für menschliche Haut, umfassend eine die Haut wirksam weichmachende und befeuchtende Menge einer oder mehrerer N-Alkoxyalkylamid-Verbindungen der allgemeinen Formel: 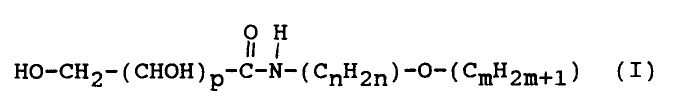 (worin p eine ganze Zahl von 1 bis 4 ist, (CₙH₂ₙ) eine geradkettige oder verzweigte Alkylenverbindung ist, worin n eine ganze Zahl von 1 bis 6 ist, und (CₘH₂ₘ₊₁) eine geradkettige oder verzweigte Alkylgruppe ist, worin m eine ganze Zahl von 1 bis 6 ist) zusammen mit einem kosmetisch annehmbaren topischen Träger.

2. Zusammensetzung nach Anspruch 1, die 1 bis 20 Gew.-% der Verbindung(en) der allgemeinen Formel (I) mit einem topischen Träger umfaßt, um z.B. eine Lotion, Creme, Salbe, einen Stift oder eine Seife zu bilden.

3. Zusammensetzung nach Anspruch 1 oder Anspruch 2, enthaltend eine oder mehrere der Verbindungen der Formel (I), worin p 4 ist.

4. Zusammensetzung nach Anspruch 1 oder Anspruch 2, enthaltend eine oder mehrere der Verbindungen der allgemeinen Formel (I), worin n 3 ist.

5. Zusammensetzung nach Anspruch 1 oder Anspruch 2, enthaltend eine oder mehrere der Verbindungen der allgemeinen Formel (I), worin m 1 ist.

6. Zusammensetzung nach einem der Ansprüche 1 bis 5, enthaltend eine oder mehrere Verbindungen der allgemeinen Formel (I), worin p 4 ist, n 3 ist und m 1 ist.

7. Zusammensetzung nach Anspruch 6, enthaltend N-Methoxypropylgluconamid.

8. Zusammensetzung nach einem der vorhergehenden Ansprüche, die 5 bis 15 Gew.-% der Verbindung(en) der allgemeinen Formel (I) enthält.

9. Zusammensetzung nach einem der vorhergehenden Ansprüche, die außer der (den) Verbindung(en) der allgemeinen Formel (I) und Wasser auch 10 bis 40 Gew.-% einer Kombination von chemisch inerten kosmetisch wirksamen Hilfskomponenten, die für diese Art von Zusammensetzung geeignet sind, enthält.

10. Zusammensetzung nach einem der vorhergehenden Ansprüche, die in Form einer Emulsion vorliegt.

11. Zusammensetzung nach Anspruch 10, die eine oder mehrere Verbindungen der allgemeinen Formel (I) enthält zusammen mit:
- 0,75 bis 7,00 Gew.-% eines oder mehrerer Emulgiermittel;
- 3,00 bis 15,00 Gew.-% eines oder mehrerer Weichmacher;
- 0,10 bis 5,00 Gew.-% eines oder mehrerer Gleitmittel;
- 0,20 bis 1,00 Gew.-% eines oder mehrerer Konservierungsmittel;
- 0,20 bis 1,00 Gew.-% Parfum und/oder
- 0,01 bis 0,10 Gew.-% eines oder mehrerer Farbstoffe, wobei der Ausgleich Wasser ist.

12. Verwendung einer oder mehrerer N-Alkoxyalkylamid-Verbindungen der allgemeinen Formel: 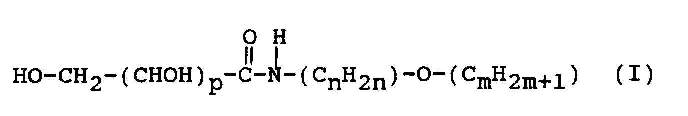 (worin p eine ganze Zahl von 1 bis 4 ist, (CₙH₂ₙ) eine geradkettige oder verzweigte Alkylenverbindung ist, worin n eine ganze Zahl von 1 bis 6 ist und (CₘH₂ₘ₊₁) eine geradkettige oder verzweigte Alkylgruppe ist, worin m eine ganze Zahl von 1 bis 6 ist) zur Herstellung einer Zusammensetzung zur Verhütung, Verbesserung oder Heilung von trockenen Hautzuständen und/oder Akne, Psoriasis, Seborrhoe, Keratose, Windelausschlag, Sonnenbrand und durch Wind geschädigte Hautstellen.

13. Verwendung nach Anspruch 12 zur Herstellung einer Zusammensetzung zur Befeuchtung und zum Weichmachen von menschlicher Haut, die eine Lotion, Creme, Salbe, ein Stift oder eine Seife ist.

14. Verwendung nach Anspruch 12 oder Anspruch 13, worin die verwendete Verbindung der allgemeinen Formel (I) Methoxypropylgluconamid ist oder einschließt.

15. Methoxypropylgluconamid.

## Patentansprüche (Patentansprüche für folgende(n) Vertragsstaat(en): ES)

1. Verfahren zur Herstellung einer kosmetischen Zubereitung zur äußeren Anwendung für menschliche Haut, worin man eine oder mehrere N-Alkoxyalkylamid-Verbindungen der allgemeinen Formel 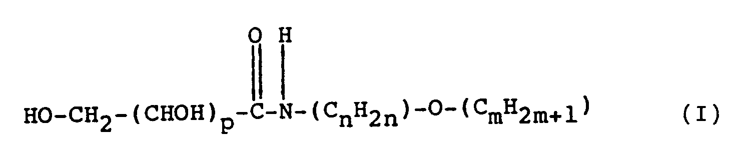 einarbeitet (worin p eine ganze Zahl von 1 bis 4 ist, (CₙH₂ₙ) eine geradkettige oder verzweigte Alkylenverbindung ist, worin n eine ganze Zahl von 1 bis 6 ist, und (CₘH₂ₘ₊₁) eine geradkettige oder verzweigte Alkylgruppe ist, worin m eine ganze Zahl von 1 bis 6 ist) in einen kosmetisch annehmbaren topischen Träger in einer ausreichenden Menge, um der resultierenden kosmetischen Zubereitung eine die Haut weichmachende und befeuchtende Wirkung zu verleihen.

2. Verfahren nach Anspruch 1, worin man 1 bis 20 Gew.-% der Verbindung(en) der allgemeinen Formel I in einen solchen topischen Träger einarbeitet, um eine Lotion, Creme, Salbe, einen Stift oder eine Seife zu bilden.

3. Verfahren nach Anspruch 1 oder Anspruch 2, worin man eine oder mehrere der Verbindungen der allgemeinen Formel I, worin p 4 ist, einarbeitet.

4. Verfahren nach Anspruch 1 oder Anspruch 2, worin man eine oder mehrere der Verbindungen der allgemeinen Formel I, worin n 3 ist, einarbeitet.

5. Verfahren nach Anspruch 1 oder Anspruch 2, worin man eine oder mehrere der Verbindungen der allgemeinen Formel I, worin m 1 ist, einarbeitet.

6. Verfahren nach einem der Ansprüche 1 bis 5, worin man eine oder mehrere der Verbindungen der allgemeinen Formel I, worin p 4 ist, n 3 ist und m 1 ist, einarbeitet.

7. Verfahren nach Anspruch 6, worin man N-Methoxypropylgluconamid einarbeitet.

8. Verfahren nach einem der vorhergehenden Ansprüche, worin man 5 bis 15 Gew.-% der Verbindung(en) der allgemeinen Formel I einarbeitet.

9. Verfahren nach einem der vorhergehenden Ansprüche, worin der kosmetisch annehmbare topische Träger Wasser enthält und 10 bis 40 Gew.-% einer Kombination von chemisch inerten, kosmetisch wirksamen Hilfskomponenten, die für diese Art der erwünschten Zusammensetzung geeignet sind.

10. Verfahren nach einem der vorhergehenden Ansprüche, worin die so hergestellte kosmetische Zubereitung in Form einer Emulsion vorliegt.

11. Verfahren nach Anspruch 10, worin die Verbindung(en) der allgemeinen Formel I in einem Träger eingearbeitet werden, der enthält:
- 0,75 bis 7,00 Gew.-% eines oder mehrerer Emulgiermittel;
- 3,00 bis 15,00 Gew.-% eines oder mehrerer Weichmacher;
- 0,10 bis 5,00 Gew.-% eines oder mehrerer Gleitmittel;
- 0,20 bis 1,00 Gew.-% eines oder mehrerer Konservierungsmittel;
- 0,20 bis 1,00 Gew.-% Parfum und/oder
- 0,01 bis 0,10 Gew.-% eines oder mehrerer Farbstoffe, wobei der Ausgleich Wasser ist.

12. Verfahren nach einem der vorhergehenden Ansprüche, enthaltend den vorhergehenden Schritt der Herstellung der N-Alkoxyalkylamid-Verbindung(en) der allgemeinen Formel I zur Einlagerung in den Träger durch Umsetzung eines Amins der allgemeinen Formel:
H₂N-(CₙH₂ₙ)-O-(CₘH₂ₘ₊₁) (III)
(worin -(CₙH₂ₙ)- eine geradkettige oder verzweigte Alkylenverbindung ist, worin n eine ganze Zahl von 1 bis 6 ist, und -(CₘH₂ₘ₊₁)- eine geradkettige oder verzweigte Alkylgruppe ist, worin m eine ganze Zahl von 1 bis 6 ist) unter Amidbildungsbedingungen entweder mit einer Carbonsäure der allgemeinen Formel: 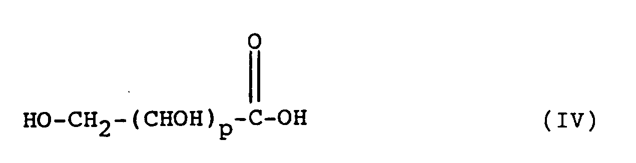 (worin p eine ganze Zahl von 1 bis 4 ist) oder mit einem Lacton der allgemeinen Formel: 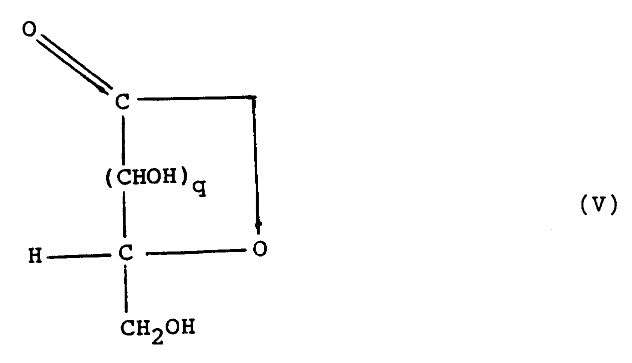 (worin q eine ganze Zahl von 1 bis 3 ist) und anschließend Einarbeiten des erwünschten Produkts in den Träger.

13. Verfahren nach Anspruch 12, worin das verwendete Amin der allgemeinen Formel III Methoxypropylamin ist.

14. Verfahren nach Anspruch 12 oder Anspruch 13, worin die verwendete Carbonsäure der allgemeinen Formel IV Gluconsäure ist.

15. Verfahren nach Anspruch 12 oder Anspruch 13, worin das verwendete Lacton der allgemeinen Formel V Glucono-delta-lacton ist.

16. Verfahren zur Herstellung von N-Methoxypropylgluconamid, worin man Methoxypropylamin unter Amidbildungsbedingungen entweder mit Gluconsäure oder mit Glucono-delta-lacton umsetzt.

17. Verfahren nach Anspruch 16, worin ein geringer molarer Überschuß von Methoxypropylamin mit Glucono-delta-lacton in Lösung in einem aliphatischen Alkohol unter Rückflußbedingungen bei Atmosphärendruck umgesetzt wird.

## Patentansprüche (Patentansprüche für folgende(n) Vertragsstaat(en): GR)

1. Hautpflege-Zusammensetzung zur äußeren Anwendung für menschliche Haut, umfassend eine die Haut wirksam weichmachende und befeuchtende Menge einer oder mehrerer N-Alkoxyalkylamid-Verbindungen der allgemeinen Formel: 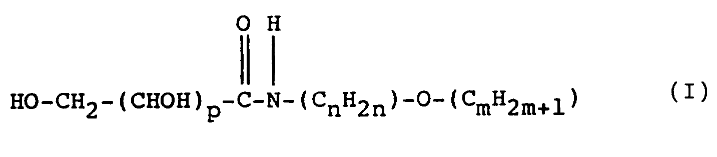 (worin p eine ganze Zahl von 1 bis 4 ist, (CₙH₂ₙ) eine geradkettige oder verzweigte Alkylenverbindung ist, worin n eine ganze Zahl von 1 bis 6 ist, und (CₘH₂ₘ₊₁) eine geradkettige oder verzweigte Alkylgruppe ist, worin m eine ganze Zahl von 1 bis 6 ist) zusammen mit einem kosmetisch annehmbaren topischen Träger.

2. Zusammensetzung nach Anspruch 1, die 1 bis 20 Gew.-% der Verbindung(en) der allgemeinen Formel I mit einem topischen Träger umfaßt, um z.B. eine Lotion, Creme, Salbe, einen Stift oder eine Seife zu bilden.

3. Zusammensetzung nach Anspruch 1 oder Anspruch 2, enthaltend eine oder mehrere der Verbindungen der allgemeinen Formel I, worin p 4 ist.

4. Zusammensetzung nach Anspruch 1 oder Anspruch 2, enthaltend eine oder mehrere der Verbindungen der allgemeinen Formel I, worin n 3 ist.

5. Zusammensetzung nach Anspruch 1 oder Anspruch 2, enthaltend eine oder mehrere der Verbindungen der allgemeinen Formel I, worin m 1 ist.

6. Zusammensetzung nach einem der Ansprüche 1 bis 5, enthaltend eine oder mehrere der Verbindungen der allgemeinen Formel I, worin p 4 ist, n 3 ist und m 1 ist.

7. Zusammensetzung nach Anspruch 6, enthaltend N-Methoxypropylgluconamid.

8. Zusammensetzung nach einem der vorhergehenden Ansprüche, die 5 bis 15 Gew.-% der Verbindung(en) der allgemeinen Formel I enthält.

9. Zusammensetzung nach einem der vorhergehenden Ansprüche, die außer der (den) Verbindung(en) der allgemeinen Formel I und Wasser auch 10 bis 40 Gew.-% einer Kombination von chemisch inerten kosmetisch wirksamen Hilfskomponenten, die für diese Art von Zusammensetzung geeignet sind, enthält.

10. Zusammensetzung nach einem der vorhergehenden Ansprüche, die in Form einer Emulsion vorliegt.

11. Zusammensetzung nach Anspruch 10, die eine oder mehrere der Verbindungen der allgemeinen Formel I enthält zusammen mit:
- 0,75 bis 7,00 Gew.-% eines oder mehrerer Emulgiermittel;
- 3,00 bis 15,00 Gew.-% eines oder mehrerer Weichmacher;
- 0,10 bis 5,00 Gew.-% eines oder mehrerer Gleitmittel;
- 0,20 bis 1,00 Gew.-% eines oder mehrerer Konservierungsmittel;
- 0,20 bis 1,00 Gew.-% Parfum und/oder
- 0,01 bis 0,10 Gew.-% eines oder mehrerer Farbstoffe, wobei der Ausgleich Wasser ist.

12. Verfahren zur Herstellung einer kosmetischen Zubereitung zur äußeren Anwendung für menschliche Haut nach einem der Ansprüche 1 bis 11, worin man eine oder mehrere der N-Alkoxyalkylamid-Verbindungen der allgemeinen Formel: 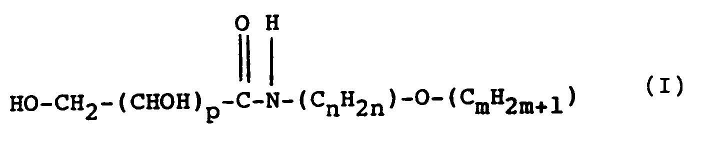 einarbeitet (worin p eine ganze Zahl von 1 bis 4 ist, (CₙH₂ₙ) eine geradkettige oder verzweigte Alkylenverbindung ist, worin n eine ganze Zahl von 1 bis 6 ist, und (CₘH₂ₘ₊₁) eine geradkettige oder verzweigte Alkylgruppe ist, worin m eine ganze Zahl von 1 bis 6 ist) in einen kosmetisch annehmbaren topischen Träger in einer ausreichenden Menge, um der resultierenden kosmetischen Zubereitung eine die Haut weichmachende und befeuchtende Wirkung zu verleihen.

13. Verfahren nach Anspruch 12, enthaltend den vorhergehenden Schritt der Herstellung der N-Alkoxyalkylamid-Verbindung(en) der allgemeinen Formel I zur Einarbeitung in den Träger duch Umsetzung eines Amins der allgemeinen Formel:
H₂N-(CₙH₂ₙ)-O-(CₘH₂ₘ₊₁) (III)
(wobei -(CₙH₂ₙ)- und -(CₘH₂ₘ₊₁)- oben definiert sind) unter Amidbildungsbedingungen entweder mit einer Carbonsäure der allgemeinen Formel: 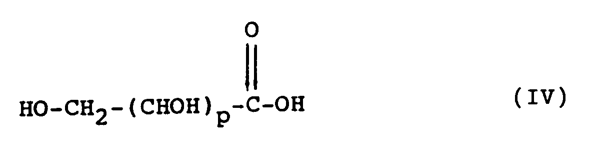 (worin p eine ganze Zahl von 1 bis 4 ist) oder mit einem Lacton der allgemeinen Formel: 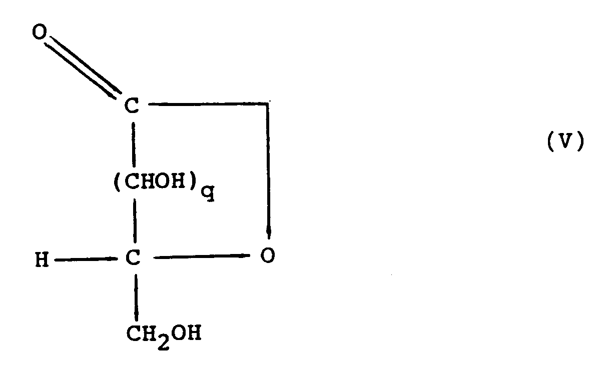 (worin q eine ganze Zahl von 1 bis 3 ist).

14. Verfahren nach Anspruch 13, worin das verwendete Amin der allgemeinen Formel III Methoxypropylamin ist.

15. Verfahren nach Anspruch 13 oder Anspruch 14, worin die verwendete Carbonsäure der allgemeinen Formel IV Gluconsäure ist.

16. Verfahren nach Anspruch 13 oder Anspruch 14, worin das verwendete Lacton der allgemeinen Formel V Glucono-delta-lacton ist.

17. Verfahren zur Herstellung von N-Methoxypropylgluconamid, worin man Methoxypropylamin unter Amidbildungsbedingungen entweder mit Gluconsäure oder mit Glucono-delta-lacton umsetzt.

18. Verfahren nach Anspruch 17, worin ein geringer molarer Überschuß von Methoxypropylamin mit Glucono-delta-lacton in Lösung in einem aliphatischen Alkohol unter Rückflußbedingungen bei Atmosphärendruck umgesetzt wird.

## Revendications (Revendications pour l'(les) Etat(s) contractant(s) suivant(s): AT, BE, CH, LI, DE, FR, GB, IT, LU, NL, SE)

1. Composition de traitement de la peau pour application topique sur la peau humaine comprenant une quantité efficace, pour hydrater et adoucir la peau, d'un ou plusieurs composés N-alkoxyalkylamides de formule générale: 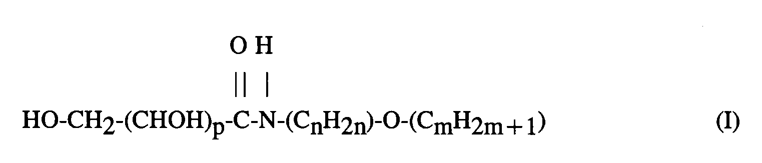 (dans laquelle p est un nombre entier de 1 à 4, (CₙH₂ₙ) est une liaison alkylène à chaîne linéaire ou ramifiée dans laquelle n est un nombre entier de 1 à 6, et (CₘH₂ₘ₊₁) est un groupe alkyle à chaîne linéaire ou ramifiée dans lequel m est un nombre entier de 1 à 6) associé à un support topique acceptable cosmétiquement.

2. Composition selon la revendication 1, qui comprend de 1 à 20% en poids du (des) composé(s) de formule générale I avec un support topique de façon à former une lotion, une crème, une pommade, un bâtonnet ou un savon.

3. Composition selon la revendication 1 ou la revendication 2, comprenant un ou plusieurs des composés de formule générale I dans laquelle p est 4.

4. Composition selon la revendication 1 ou la revendication 2, comprenant un ou plusieurs des composés de formule générale I dans laquelle n est 3.

5. Composition selon la revendication 1 ou la revendication 2, comprenant un ou plusieurs des composés de formule générale I dans laquelle m est 1.

6. Composition selon l'une quelconque des revendications 1 à 5, comprenant un ou plusieurs des composés de formule générale I dans laquelle p est 4, n est 3 et m est 1.

7. Composition selon la revendication 6, comprenant du N-méthoxy-propyl-gluconamide.

8. Composition selon l'une quelconque des revendications précédentes, qui contient de 5% à 15% en poids du (des) composé(s) de formule générale I.

9. Composition selon l'une quelconque des revendications précédentes, qui en plus du (des) composé(s) de formule générale I et de l'eau contient également de 10% à 40% en poids d'une combinaison de composants auxiliaires efficaces cosmétiquement, inertes chimiquement, appropriés au type de composition.

10. Composition selon l'une quelconque des revendications précédentes, qui est sous la forme d'une émulsion.

11. Composition selon la revendication 10, qui contient un ou plusieurs des composés de formule générale I, en association avec:
- de 0,75% à 7,00% en poids d'un ou plusieurs agent(s) émulsifiant(s);
- de 3,00% à 15,00% en poids d'un ou plusieurs émollient(s);
- de 0,10% à 5,00% en poids d'un ou plusieurs lubrifiant(s);
- de 0,20% à 1,00% en poids d'un ou plusieurs agent(s) de conservation;
- de 0,20% à 1,00% en poids de parfum;
- et/ou de 0,01% à 0,10% en poids d'un ou plusieurs colorant(s), le complément étant de l'eau.

12. Utilisation d'un ou plusieurs composés N-alkoxyalkylamides de formule générale: 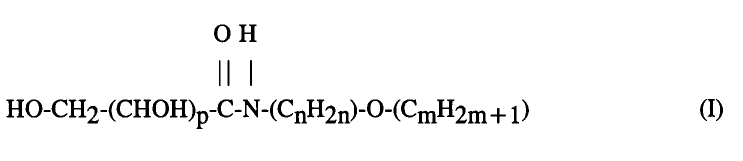 (dans laquelle p est un nombre entier de 1 à 4, (CₙH₂ₙ) est une liaison alkylène à chaîne ramifiée ou linéaire dans laquelle n est un nombre entier de 1 à 6, et (CₘH₂ₘ₊₁) est un groupe alkyle à chaîne ramifiée ou linéaire dans lequel m est un nombre entier de 1 à 6) pour la préparation d'une composition pour la prévention, l'amélioration ou le traitement des conditions de peaux sèches et/ou de l'acné, du psoriasis, de la séborrhée, de la kératose, de l'érythème provoque par les couches, des brûlures de soleil et de vent.

13. Utilisation selon la revendication 12, pour la préparation d'une composition pour hydrater et adoucir la peau humaine, se présentant sous forme de lotion, de crème, de pommade, de bâtonnet ou de savon.

14. Utilisation selon la revendication 12 ou la revendication 13, dans laquelle le composé de formule générale I utilisé est ou comprend le méthoxy-propyl-gluconamide.

15. Méthoxy-propyl-gluconamide

## Revendications (Revendications pour l'(les) Etat(s) contractant(s) suivant(s): ES)

1. Méthode de fabrication d'une préparation cosmétique pour application topique sur la peau humaine dans laquelle on incorpore un ou plusieurs composés N-alkoxyalkylamides de formule générale: 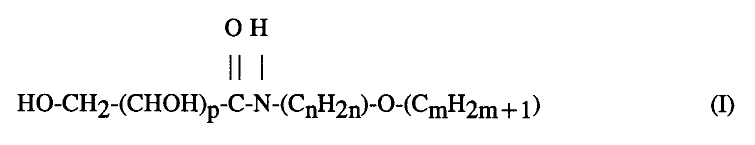 (dans laquelle p est un nombre entier de 1 à 4, (CₙH₂ₙ) est une liaison alkylène à chaîne linéaire ou ramifiée dans laquelle n est un nombre entier de 1 à 6, et (CₘH₂ₘ₊₁) est un groupe alkyle à chaîne linéaire ou ramifiée dans lequel m est un nombre entier de 1 à 6) dans un support topique cosmétiquement acceptable en quantité suffisante pour conférer un effet adoucissant et hydratant de la peau à la préparation cosmétique résultante.

2. Méthode selon la revendication 1, dans laquelle on incorpore de 1 à 20% en poids de(s) composé(s) de formule générale I dans un tel support topique de façon à former une lotion, une crème, une pommade, un bâtonnet ou un savon.

3. Méthode selon la revendication 1 ou la revendication 2, dans laquelle on incorpore un ou plusieurs des composés de formule générale I dans laquelle p est 4.

4. Méthode selon la revendication 1 ou la revendication 2, dans laquelle on incorpore un ou plusieurs des composés de formule générale I dans laquelle n est 3.

5. Méthode selon la revendication 1 ou la revendication 2, dans laquelle on incorpore un ou plusieurs des composés de formule générale I dans laquelle m est 1.

6. Méthode selon l'une des revendications 1 à 5, dans laquelle on incorpore un ou plusieurs des composés de formule générale I dans laquelle p est 4, n est 3 et m est 1.

7. Méthode selon la revendication 6, dans laquelle on incorpore du N-méthoxy-propyl-gluconamide.

8. Méthode selon l'une quelconque des revendications précédentes, dans laquelle on incorpore de 5% à 15% en poids du (des) composé(s) de formule générale I.

9. Méthode selon l'une quelconque des revendications précédentes, dans laquelle le support topique cosmétiquement acceptable contient de l'eau, et de 10% à 40% en poids d'une combinaison de composants auxiliaires efficaces cosmétiquement, inertes chimiquement, appropriés au type de composition désirée.

10. Méthode selon l'une quelconque des revendications précédentes, dans laquelle la préparation cosmétique ainsi préparée prend la forme d'une émulsion.

11. Méthode selon la revendication 10, dans laquelle le(s) composé(s) de formule générale I sont incorporés dans un support qui contient:
- de 0,75% à 7,00% en poids d'un ou plusieurs agent(s) émulsifiant(s);
- de 3,00% à 15,00% en poids d'un ou plusieurs émollient(s);
- de 0,10% à 5,00% en poids d'un ou plusieurs lubrifiant(s);
- de 0,20% à 1,00% en poids d'un ou plusieurs agent(s) de conservation;
- de 0,20% à 1,00% en poids de parfum;
- et/ou de 0,01% à 0,10% en poids d'un ou plusieurs colorant(s), le complément étant de l'eau.

12. Méthode selon l'une des revendications précédentes, qui comporte l'étape préliminaire de préparation de(s) composé(s) N-alkoxyalkylamide(s) de formule générale I pour l'incorporer dans le support en faisant réagir une amine de formule générale:
H₂N-(CₙH₂ₙ)-O-(CₘH₂ₘ₊₁) (III)
(dans laquelle -(CₙH₂ₙ)- est un groupe alkylène à chaîne ramifiée ou linéaire dans laquelle n est un nombre entier de 1 à 6, et -(CₘH₂ₘ₊₁) est un groupe alkyle à chaîne ramifiée ou linéaire dans lequel m est un nombre entier de 1 à 6), dans des conditions permettant la formation d'amide soit avec un acide carboxylique ayant la formule générale: 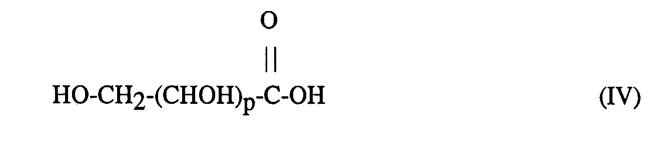 (dans laquelle p est un nombre entier de 1 à 4) soit avec une lactone de formule générale: 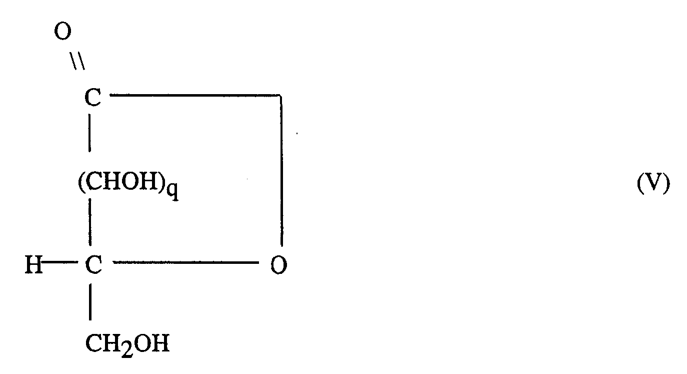 (dans laquelle q est un nombre entier de 1 à 3), et ensuite l'incorporation du produit désiré dans le support.

13. Méthode selon la revendication 12, dans laquelle l'amine de formule générale III utilisée est la méthoxypropylamine.

14. Méthode selon la revendication 12 ou la revendication 13, dans laquelle l'acide carboxylique de formule générale IV utilisé est l'acide gluconique.

15. Méthode selon la revendication 12 ou la revendication 13, dans laquelle la lactone de formule générale V utilisée est la glucono-delta-lactone.

16. Méthode pour la préparation de N-méthoxy-propylgluconamide dans laquelle on fait réagir la méthoxy-propyl-amine dans des conditions permettant la formation d'amide soit avec de l'acide gluconique, soit avec de la glucono-delta-lactone.

17. Méthode selon la revendication 16, dans laquelle un léger excès molaire de méthoxy-propyl-amine est mis à réagir avec de la glucono-delta-lactone en solution dans un alcool aliphatique au reflux à pression atmosphérique.

## Revendications (Revendications pour l'(les) Etat(s) contractant(s) suivant(s): GR)

1. Composition de traitement de la peau pour application topique sur la peau humaine comprenant une quantité efficace, pour hydrater et adoucir la peau, d'un ou plusieurs composés N-alkoxyalkylamides de formule générale: 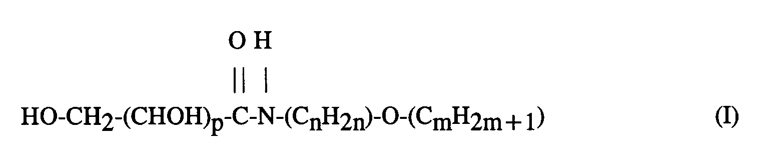 (dans laquelle p est un nombre entier de 1 à 4, (CₙH₂ₙ) est une liaison alkylène à chaîne linéaire ou ramifiée dans laquelle n est un nombre entier de 1 à 6, et (CₘH₂ₘ₊₁) est un groupe alkyle à chaîne linéaire ou ramifiée dans lequel m est un nombre entier de 1 à 6) associé à un support topique acceptable cosmétiquement.

2. Composition selon la revendication 1, qui comprend de 1 à 20% en poids du (des) composé(s) de formule générale I avec un support topique de façon à former une lotion, une crème, une pommade, un bâtonnet ou un savon.

3. Composition selon la revendication 1 ou la revendication 2, comprenant un ou plusieurs des composés de formule générale I dans laquelle p est 4.

4. Composition selon la revendication 1 ou la revendication 2, comprenant un ou plusieurs des composés de formule générale I dans laquelle n est 3.

5. Composition selon la revendication 1 ou la revendication 2, comprenant un ou plusieurs des composés de formule générale I dans laquelle m est 1.

6. Composition selon l'une quelconque des revendications 1 à 5, comprenant un ou plusieurs des composés de formule générale I dans laquelle p est 4, n est 3 et m est 1.

7. Composition selon la revendication 6, comprenant du N-méthoxy-propyl-gluconamide.

8. Composition selon l'une quelconque des revendications précédentes, qui contient de 5% à 15% en poids du (des) composé(s) de formule générale I.

9. Composition selon l'une quelconque des revendications précédentes, qui en plus du (des) composé(s) de formule générale I et de l'eau, contient également de 10% à 40% en poids d'une combinaison de composants auxiliaires efficaces cosmétiquement, inertes chimiquement, appropriés au type de composition.

10. Composition selon l'une quelconque des revendications précédentes qui est sous la forme d'une émulsion.

11. Composition selon la revendication 10, qui contient un ou plusieurs des composés de formule générale I, en association avec:
- de 0,75% à 7,00% en poids d'un ou plusieurs agent(s) émulsifiant(s);
- de 3,00% à 15,00% en poids d'un ou plusieurs émollient(s);
- de 0,10% à 5,00% en poids d'un ou plusieurs lubrifiant(s);
- de 0,20% à 1,00% en poids d'un ou plusieurs agent(s) de conservation;
- de 0,20% à 1,00% en poids de parfum;
- et/ou de 0,01% à 0,10% en poids d'un ou plusieurs colorant(s), le complément étant de l'eau.

12. Méthode de fabrication d'une préparation cosmétique pour application topique sur la peau humaine selon l'une quelconque des revendications 1 à 11, dans laquelle on incorpore un ou plusieurs composés N-alkoxyalkylamides de formule générale: 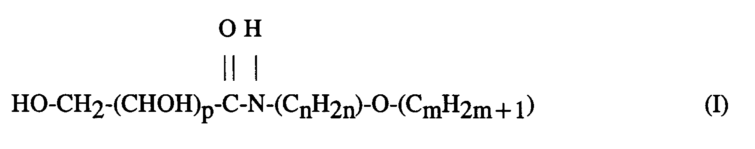 (dans laquelle p est un nombre entier de 1 à 4, (CₙH₂ₙ) est un groupe alkylène à chaîne ramifiée ou linéaire dans laquelle n est un nombre entier de 1 à 6, et (CₘH₂ₘ₊₁) est un groupe alkyle à chaîne ramifiée ou linéaire dans lequel m est un nombre entier de 1 à 6), dans un support topique cosmétiquement acceptable en quantité suffisante pour conférer un effet adoucissant et hydratant de la peau à la préparation cosmétique résultante.

13. Méthode selon la revendication 12, qui comporte l'étape préliminaire de préparation de(s) composé(s) N-alkoxyalkylamide(s) de formule générale I pour l'incorporer dans le support en faisant réagir une amine de formule générale:
H₂N-(CₙH₂ₙ)-O-(CₘH₂ₘ₊₁) (III)
(dans laquelle (CₙH₂ₙ) et (CₘH₂ₘ₊₁) sont comme définis précédemment) dans des conditions permettant la formation d'amide soit avec un acide carboxylique ayant la formule générale: 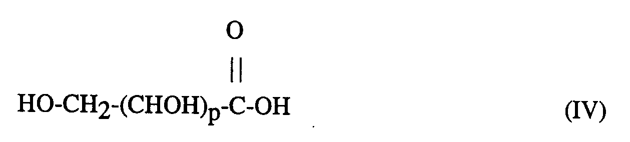 (dans laquelle p est un nombre entier de 1 à 4) soit avec une lactone de formule générale: 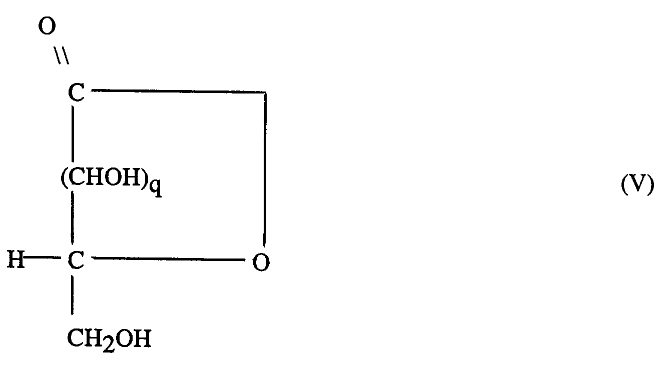 (dans laquelle q est un nombre entier de 1 à 3).

14. Méthode selon la revendication 13, dans laquelle l'amine de formule générale III utilisée est la méthoxypropylamine.

15. Méthode selon la revendication 13 ou la revendication 14, dans laquelle l'acide carboxylique de formule générale IV utilisé est l'acide gluconique.

16. Méthode selon la revendication 13 ou la revendication 14, dans laquelle la lactone de formule générale V utilisée est la glucono-delta-lactone.

17. Méthode pour la préparation de N-méthoxy-propylgluconamide dans laquelle on fait réagir le méthoxy-propyl-amine dans des conditions permettant la formation d'amide soit avec de l'acide gluconique, soit avec de la glucono-delta-lactone.

18. Méthode selon la revendication 17, dans laquelle un léger excès molaire de méthoxy-propyl-amine est mis à réagir avec de la glucono-delta-lactone en solution dans un alcool aliphatique au reflux à pression atmosphérique.
